# FASCICULE DE BREVET EUROPEEN

(11) **EP 0 572 339 B1**
(45) Date de publication et mention de la délivrance du brevet: **04.12.1996**
(21) Numéro de dépôt: 93420212.8
(22) Date de dépôt: 25.05.1993
(51) Int. Cl.: A61F 2/42

(54) **Prothèse totale pour l'articulation métacarpo-phalangienne**
Gesamtprothese für das Metakarpophalangealgelenk
Total prosthesis for the metacarpal-phalangeal joint

(30) Priorité: 25.05.1992 FR 9206588
(43) Date de publication de la demande: 01.12.1993
(73) Titulaire: TORNIER SA, F-38330 Saint Ismier (FR)
(72) Inventeur: Tornier, Alain, F-38330 Saint-Ismier (FR)
(74) Mandataire: Karmin, Roger

(56) Documents cités:
- FR-A- 2 269 324
- FR-A- 2 668 703
- US-A- 4 242 759
- US-A- 4 731 087

## Description

La présente invention a pour objet une prothèse totale pour l'articulation métacarpo-phalangienne de chaque doigt d'une main, qui comprend deux éléments distincts prenant contact l'un avec l'autre par l'intermédiaire de la structure osseuse, ligamentaire ou musculaire restant en place.

On connaît des prothèses de ce genre qui comprennent généralement un bloc de matériau élastique implanté dans les diaphyses osseuses pour remplacer la partie de l'articulation métacarpo-phalangienne endommagée. Ces blocs sont conformés de manière à réduire le plus possible les contraintes dues au frottement, de manière que le mouvement de la prothèse soit alors basé sur les caractéristiques élastiques du matériau employé.

De telles prothèses comportent certains inconvénients en ce qui concerne tant la durée de vie des blocs élastiques, notablement courte, que les contraintes que ces blocs engendrent sur l'articulation du fait de leur conception rudimentaire.

On connaît aussi des prothèses métalliques qui assimilent l'articulation métacarpo-phalangienne à une articulation du type charnière. Ces prothèses ne sont pas susceptibles de reproduire parfaitement la cinématique normale de l'articulation, ce qui entraîne des complications importantes. Ces complications sont généralement dues aux contraintes que la prothèse engendre sur l'articulation et qui se traduisent par un descellement du corps de la prothèse.

On connaît également d'après le brevet américain N° 4 242 759 une prothèse totale pour l'articulation métacarpophalangienne du genre comprenant un premier élément pourvu d'une tige pour son ancrage dans la diaphyse phalangienne de l'os et d'une tête évasée et un second élément constitué d'une tige pour son ancrage dans la diaphyse métacarpienne de l'os et d'une tête coopérant avec celle du premier élément.

On constate que les rayons de courbures de chacune des têtes ne permettent pas une parfaite reproduction du mouvement de l'articulation métacarpo-phalangienne.

C'est à ces inconvénients qu'entend remédier principalement la présente invention, et ce à l'aide d'une prothèse qui est susceptible de reproduire de manière parfaite le mouvement de l'articulation métacarpophalangienne.

La prothèse totale suivant l'invention est décrit dans la revendication 1.

Un avantage de l'invention consiste à réaliser une prothèse totale non contrainte qui permet à l'ensemble de la structure osseuse ou ligamentaire ou musculaire restant en place de guider le mouvement de l'articulation métacarpo-phalangienne.

La prothèse totale de l'articulation métacarpo-phalangienne est utilisable avec ou sans ciment. En outre, les deux éléments constituant la prothèse totale présentent respectivement des surfaces articulaires qui comportent plusieurs rayons de courbure et de profils distincts, de manière que la cinématique diffère suivant les positions angulaires des deux éléments l'un par rapport à l'autre.

Le dessin annexé, donné à titre d'exemple, permettra de mieux comprendre l'invention, les caractéristiques qu'elle présente et les avantages qu'elle est susceptible de procurer :
Fig. 1 est une vue d'ensemble représentant les os constituant une main droite dont l'une des articulations métacarpo-phalangiennes est réalisée par une prothèse totale suivant l'invention.
Fig. 2 est une vue latérale avec coupe partielle illustrant l'agencement de la prothèse totale en position d'extension.
Fig. 3 est une coupe partielle agrandie suivant III-III de fig. 2, montrant les surfaces articulaires des deux éléments qui sont en contact en position d'extension.
Fig. 4 est une vue de dessus montrant la prothèse totale dans la même position que celle suivant fig. 1.
Fig. 5 est une vue latérale semblable à celle de fig. 1, mais montrant la prothèse totale dans une position de flexion phalangienne inclinée d'un angle de 30° vers le bas par rapport à un plan horizontal.
Fig. 6 est une coupe partielle agrandie suivant VI-VI de fig. 5 montrant les surfaces articulaires des deux éléments qui sont en contact en position de flexion inclinée suivant fig. 5.
Fig. 7 est une vue latérale avec coupe partielle représentant la prothèse totale dans une position de flexion phalangienne inclinée d'un angle de 90° par rapport à un plan horizontal.
Fig. 8 est une coupe partielle agrandie suivant VIII-VIII de fig. 7 montrant les surfaces articulaires des deux éléments qui sont en contact en position de flexion inclinée suivant fig. 7.

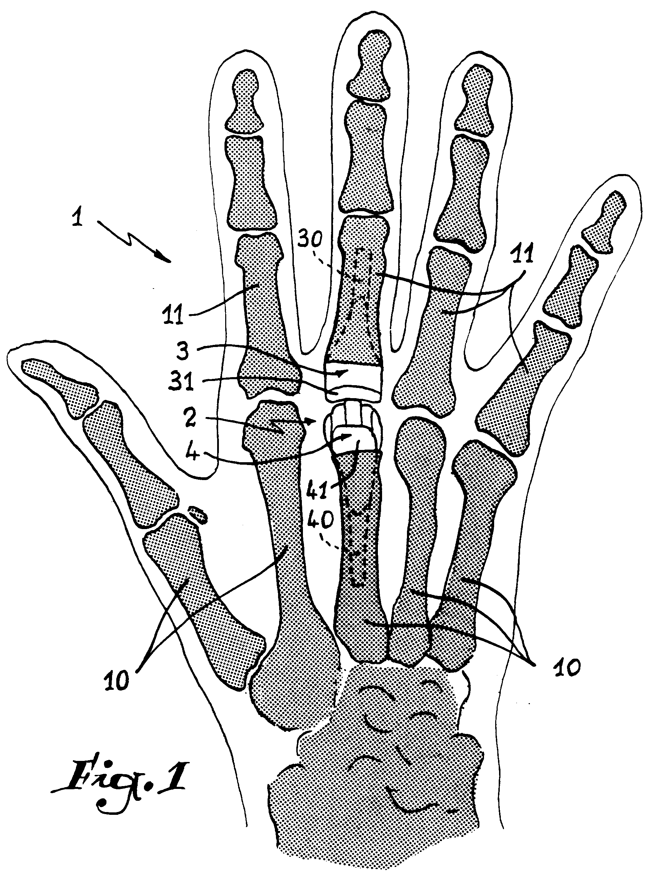

On a représenté en fig. 1 une main droite 1 dont les articulations métacarpo-phalangiennes sont constituées par des os métacarpiens 10 et des phalanges 11, l'une de celles-ci étant réalisé par l'intermédiaire d'une prothèse totale 2.

La prothèse totale comprend un premier élément 3 et un second élément 4 qui sont ancrés respectivement dans la diaphyse phalangienne et métacarpienne. Ces deux éléments sont indépendants l'un de l'autre et sont en contact uniquement par l'intermédiaire de leurs surfaces articulaires que l'on distinguera mieux plus loin.

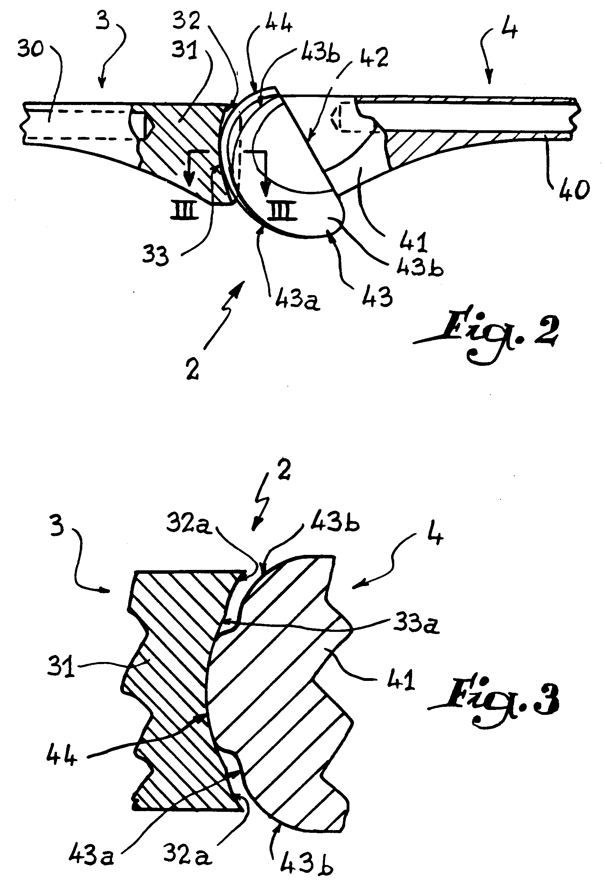

Comme illustré en fig. 2, le premier élément 3 de la prothèse comporte une tige creuse 30 présentant un profil en forme de tulipe pour permettre son ancrage dans la diaphyse phalangienne. Cette tige 30 est adaptée à la morphologie interne de l'os 11 et peut être pourvue d'un dispositif visant à promouvoir un ancrage par repousse osseuse.

A l'une de ses extrémités, la tige 30 est pourvue d'une tête évasée 31 formant la partie articulaire de la phalange. Cette tête évasée 31 comporte deux surfaces articulaires distinctes 32 et 33 à profil concave de manière à reproduire les surfaces de l'os phalangien 11. Les surfaces articulaires 32, 33 présentent respectivement un rayon de courbures 32a et 33a en forme de tonneau. La tête évasée 31 peut être par exemple rapportée sur la tige phalangienne 30 par l'intermédiaire d'une goupille (non représentée) de manière à s'adapter à toutes les morphologies d'une articulation métacarpo-phalangienne.

Le second élément 4 comprend une tige creuse 40 présentant un profil en forme de tulipe pour permettre son ancrage à l'intérieur de la diaphyse métacarpienne de l'os 10. Cette tige 40 est adaptée à la morphologie interne de l'os 10 et peut comprendre un dispositif visant à promouvoir un ancrage par repousse osseuse.

A l'une des extrémités de la tige 40 est prévue une tête 41 qui présente une forme extérieure ayant pour qualité de s'adapter parfaitement, dans tous les plans, au profil prévu sur la tête évasée 31 du premier élément 30. La tête 41 comprend, du côté de la tige 40 et de part et d'autre de celle-ci, un pan incliné 42 servant d'appui à la coupe osseuse ménagée sur la surface articulaire métacarpienne. La forme extérieure de la tête 41 se divise en deux surfaces articulaires distinctes 43 et 44 qui viennent en contact avec les surfaces articulaires 32 et 33 de la tête phalangienne 31 de manière à s'adapter aux différentes positions angulaires que peut prendre la prothèse totale 2.

La surface articulaire référencée 43 présente un rayon de courbure du type cylindrique 43a dont les bords sont arrondis suivant un profil 43b. La surface articulaire référencée 44 est disposée au milieu de la première surface 43 et présente un rayon de courbure ayant comme profil extérieur une crête en portion de sphère. La surface articulaire 44 prend naissance dans la partie inférieure de la tête 41 et au milieu du rayon de courbure 43a. Dans la partie supérieure de la tête 41, la surface articulaire 44 en forme de crête divise en deux portions équivalentes la surface articulaire 43, de manière à ne laisser apparaître que les bords ayant un profil arrondi 43b.

En fig. 2, 3 et 4, on a représenté la prothèse totale 2 en position d'extension maximale, c'est-à-dire lorsque la main 1 est par exemple posée à plat sur une surface horizontale ou verticale.

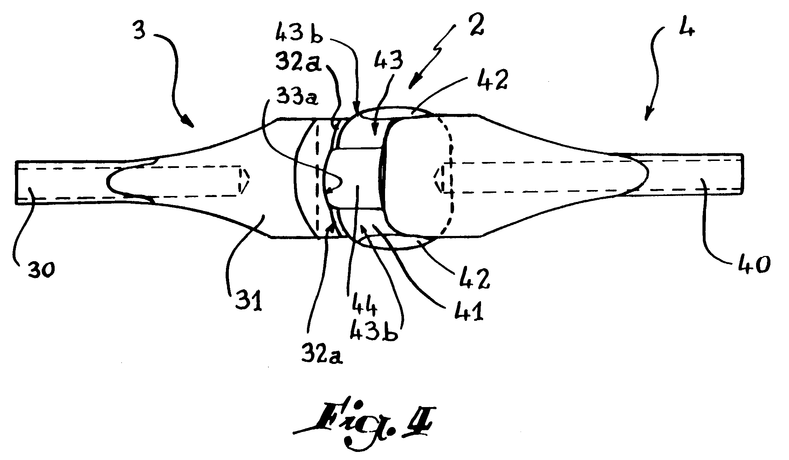

Ainsi, en fig. 3, on a montré en détail les surfaces articulaires du premier élément 3 et du second élément 4 qui viennent en contact l'une avec l'autre. On remarque que dans cette position d'extension, seule la surface articulaire 44 ayant comme profil extérieur une crête en portion de sphère et appartenant au second élément 4 est en contact avec le rayon de courbure 33a de la surface 33 du premier élément 3.

On note aussi que dans cette position le premier élément 3, c'est-à-dire celui comportant la tige phalangienne 30, peut se déplacer latéralement par rapport au second élément 4 qui est fixé dans la diaphyse métacarpienne de l'os 10.

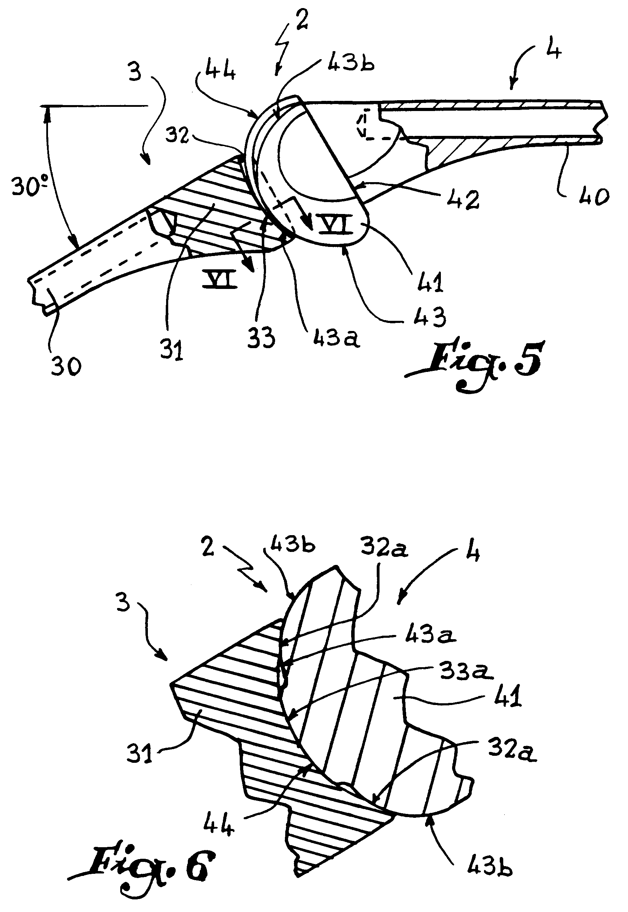

En fig. 5 et 6, on a représenté la prothèse totale 2 au début de sa flexion, c'est-à-dire lorsque l'élément 3 est incliné d'un angle de 30° par rapport au second élément 4 qui est prévu dans un plan horizontal. On constate que dans cette position les rayons de courbure 32a et 33a de l'élément 3 sont en contact respectivement avec les bords arrondis 43b de la surface articulaire 43 et d'autre part avec la surface articulaire 44.

On observera également que le début de la surface articulaire 44 en forme de crête sphérique est réalisé en harmonie avec le profil de la surface articulaire 43 pour ne pas gêner les mouvements de flexion et d'extension du premier élément 3 par rapport au second élément 4. Dans cette position inclinée à 30°, les déplacements latéraux du premier élément 3 sont limités, étant donné que le rayon de courbure 32a commence à prendre appui sur les bords arrondis 43b de la surface articulaire 43.

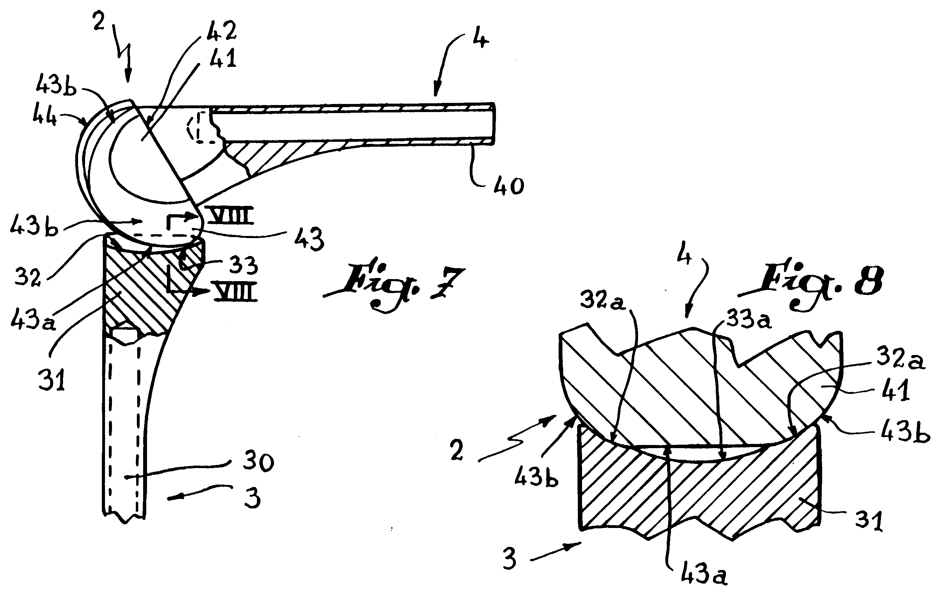

En fig. 7 et 8, la prothèse 2 est représentée en flexion totale, c'est-à-dire lorsque le premier élément 3 se trouve incliné d'un angle de 90° par rapport au second élément 4 qui est prévu dans un plan horizontal. Dans cette position, le rayon de courbure 32a de la surface articulaire 32 du premier élément 3 est uniquement en contact avec la surface articulaire 43 et plus particulièrement avec les bords arrondis 43b du rayon de courbure de type cylindrique 43a, empêchant ainsi tout déplacement latéral du premier élément 3 par rapport au second élément 4.

On note que la prothèse totale permet, grâce à ses deux surfaces articulaires 43 et 44 entrant successivement en fonction au cours du mouvement du premier élément 3 par rapport au second élément 4, de reproduire parfaitement les mouvements enregistrés dans une articulation métacarpo-phalangienne saine.

## Revendications

1. Prothèse totale pour l'articulation métacarpo-phalangienne, du genre comprenant un premier élément (3) pourvu d'une tige (30) pour son ancrage dans la diaphyse phalangienne de l'os (11) et d'une tête évasée (31) et un second élément constitué d'une tige (40) pour son ancrage dans la diaphyse métacarpienne de l'os et d'une tête coopérant à celle du premier élément, caractérisée en ce que le premier élément (3) comporte une tête évasée (31) pourvue d'au moins deux surfaces articulaires (32, 33) à profil concave présentant chacune un rayon de courbure distinct (32a et 33a) en forme de tonneau, tandis que le second élément (4) présente une tête (41) comportant un profil extérieur ayant au moins deux surfaces articulaires (43, 44) distinctes présentant respectivement un premier rayon de courbure (43a) de type cylindrique dont les bords sont arrondis suivant un profil (43b), et un second rayon de courbure (44) en forme de crête en portion de sphère prenant naissance dans la partie inférieure de ladite tête (41) et au milieu du premier rayon de courbure (43a).

2. Prothèse suivant la revendication 1, caractérisée en ce que la tige (30), prévue creuse et à profil extérieur évasé en forme de tulipe, est adaptée à la morphologie interne de l'os (11) pour améliorer l'ancrage dans la diaphyse phalangienne.

3. Prothèse suivant la revendication 1, caractérisée en ce que la tête évasée (31) est rapportée sur la tige (30) pour s'adapter à toutes les morphologies d'une articulation métacarpo-phalangienne.

4. Prothèse suivant la revendication 1, caractérisée en ce que la tige (40), prévue creuse et à profil extérieur évasé en forme de tulipe, est adaptée à la morphologie interne de l'os (10) pour améliorer l'ancrage dans la diaphyse métacarpienne.

5. Prothèse suivant la revendication 1, caractérisée en ce que la tête (41) comprend, du côté de la tige (40) et de part et d'autre de celle-ci un pan incliné (42), servant d'appui à la coupe osseuse ménagée sur la surface articulaire de l'os métacarpien (10).

6. Prothèse suivant la revendication 1, caractérisée en ce que dans la partie supérieure de la tête (41), la surface articulaire (44) en forme de crête divise en deux portions équivalentes la surface articulaire (43) de manière à ne laisser apparaître que les bords ayant un profil arrondi (43b).

7. Prothèse suivant la revendication 1, caractérisée en ce que la naissance de la surface articulaire (44) est réalisée en harmonie avec le profil de la surface articulaire (43) pour améliorer les mouvements de flexion et d'extension du premier élément (3) par rapport au second élément (4).

## Claims

1. Total prosthesis for the metacarpal-phalangeal joint, of the type comprising a first element (3) which is provided with a rod (30), for anchoring it in the phalangeal diaphysis of the bone (11), and with a flared head (31), and a second element consisting of a rod (40), for anchoring it in the metacarpal diaphysis of the bone, and of a head cooperating with that of the first element, characterized in that the first element (3) includes a flared head (31) provided with at least two articular surfaces (32, 33) of concave profile which each present a distinct radius of curvature (32a and 33a) in a barrel shape, while the second element (4) has a head (41) which includes an outer profile having at least two distinct articular surfaces (43, 44), presenting, respectively, a first radius of curvature (43a) of a cylindrical type, the edges of which are rounded in a profile (43b), and a second radius of curvature (44) in the form of a crest of a sphere portion originating in the lower part of the said head (41) and at the middle of the first radius of curvature (43a).

2. Prosthesis according to Claim 1, characterized in that the rod (30), which is made hollow and with an outer profile flared in a tulip shape, is adapted to the internal morphology of the bone (11) in order to improve the anchoring in the phalangeal diaphysis.

3. Prosthesis according to Claim 1, characterized in that the flared head (31) is attached to the rod (30) so as to adapt to all the morphologies of a metacarpal-phalangeal joint.

4. Prosthesis according to Claim 1, characterized in that the rod (40), which is made hollow and with an outer profile flared in a tulip shape, is adapted to the internal morphology of the bone (10) in order to improve the anchoring in the metacarpal diaphysis.

5. Prosthesis according to Claim 1, characterized in that the head (41) comprises, towards the rod (40), and on either side thereof, an inclined face (42) serving as a bearing for the osseous cut made on the articular surface of the metacarpal bone (10).

6. Prosthesis according to Claim 1, characterized in that, in the upper part of the head (41), the crest-shaped articular surface (44) divides the articular surface (43) into two equivalent portions in such a way as to expose only the edges having a rounded profile (43b).

7. Prosthesis according to Claim 1, characterized in that the origin of the articular surface (44) is formed in harmony with the profile of the articular surface (43) in order to improve the movements of flexion and extension of the first element (3) in relation to the second element (4).

## Patentansprüche

1. Totalprothese für das Fingergrundgelenk von der Art, die ein erstes Element (3), das mit einem Schaft (30) zu seiner Verankerung in der Phalanxdiaphyse des Knochens (11) und mit einem sich aufweitenden Kopf (31) versehen ist, und ein zweites Element aufweist, das einen Schaft (40) für seine Verankerung in der Metacarpaldiaphyse des Knochens und einen mit dem des ersten Elements zusammenwirkenden Kopf aufweist,
dadurch **gekennzeichnet**,
daß das erste Element (3) einen sich aufweitenden Kopf (31) aufweist, der mit mindestens zwei Gelenkoberflächen (32, 33) mit konkavem Profil versehen ist, von denen jede einen unterschiedlichen tonnenförmigen Krümmungsradius bzw. -bereich (32a und 33a) aufweist,
während das zweite Element (4) einen Kopf (41) aufweist, der ein Außenprofil mit mindestens zwei unterschiedlichen Gelenkoberflächen (43, 44) aufweist, die entsprechend einen ersten Krümmungsradius bzw. -bereich (43a) von zylindrischer Art, dessen Randbereiche gemäß einem Profil (43b) abgerundet sind, und einen zweiten Krümmungsradius bzw. -bereich (44) in Form eines sphärenabschnittsförmigen Steges bzw. einer solchen Wölbung aufweist, der im unteren Bereich des Kopfes (41) und mittig im ersten Krümmungsbereich (43a) entspringt.

2. Prothese nach Anspruch 1,
dadurch **gekennzeichnet**,
daß der Schaft (30), der hohl und mit einem sich aufweitenden Außenprofil in Tulpenform vorgesehen ist, der inneren Morphologie des Knochens (11) zur Verbesserung der Verankerung in der Phalarixdiaphyse angepaßt ist.

3. Prothese nach Anspruch 1,
dadurch **gekennzeichnet**,
daß der sich aufweitende Kopf (31) an den Schaft (30) zu seiner Anpassung an jegliche Morphologie eines Fingergrundgelenks angestückt ist.

4. Prothese nach Anspruch 1,
dadurch **gekennzeichnet**,
daß der Schaft (40), der hohl und mit einem sich aufweitenden Außenprofil in Tulpenform vorgesehen ist, der inneren Morphologie des Knochens (10) zur Verbesserung der Verankerung in der Metacarpaldiaphyse angepaßt ist.

5. Prothese nach Anspruch 1,
dadurch **gekennzeichnet**,
daß der Kopf (41) beidseits des Schaftes (40) eine geneigte Fläche (42) aufweist, die der auf der Gelenkfläche des Metacarpalkmochens (10) angebrachten Schnittfläche als Auflager dient.

6. Prothese nach Anspruch 1,
dadurch **gekennzeichnet**,
daß die Gelenkfläche (44) in Form eines Steges bzw.einer Wölbung die beiden äquivalenten Abschnitte der Gelenkfläche (43) im oberen Bereich des Kopfes (41) derart unterteilt, daß lediglich die Randbereiche mit einem abgerundeten Profil (43b) sichtbar sind.

7. Prothese nach Anspruch 1,
dadurch **gekennzeichnet**,
daß der Ursprung der Gelenkoberfläche (44) zur Verbesserung der Beuge- und Streckbewegung des ersten Elements (3) bezüglich des zweiten Elements (4) harmonisch bzw. übergangslos zum Profil der Gelenkoberfläche (43) gestaltet ist.
